# EUROPÄISCHE PATENTSCHRIFT

(11) **EP 1 341 451 B1**
(45) Veröffentlichungstag und Bekanntmachung des Hinweises auf die Patenterteilung: **26.05.2004**
(21) Anmeldenummer: 01978443.8
(22) Anmeldetag: 20.10.2001
(51) Int. Cl.: A61B 17/15, A61B 19/00

(54) **VERFAHREN UND VORRICHTUNG ZUR BESTIMMUNG DER MECHANISCHEN ACHSE EINES FEMURS**
METHOD AND DEVICE FOR DETERMINING THE MECHANICAL AXIS OF A FEMUR
PROCEDE ET DISPOSITIF POUR LA DETERMINATION DE L'AXE MECANIQUE D'UN FEMUR

(30) Priorität: 15.12.2000 DE 10062580
(43) Veröffentlichungstag der Anmeldung: 10.09.2003
(73) Patentinhaber: Aesculap AG & Co. KG, 78532 Tuttlingen (DE)
(72) Erfinder: LEITNER, François, F-38410 Uriage (FR)
(74) Vertreter: Böhme, Ulrich, Dr. Dipl.-Phys.
(86) Internationale Anmeldenummer: PCT/EP2001/012141
(87) Internationale Veröffentlichungsnummer: WO 2002/047559

(56) Entgegenhaltungen:
- WO-A-00/48507
- WO-A-98/40037
- US-A- 5 611 353

## Beschreibung

Die Erfindung betrifft ein Verfahren zur Bestimmung der mechanischen Achse eines Femurs, bei dem man den Femur um das Hüftgelenk bewegt, die Bewegung des Femurs mittels eines Markierelementes am Femur über ein Navigationssystem verfolgt, daraus gewonnene Positionsdaten des Femurs speichert und aus den unterschiedlichen Positionsdaten des Femurs in unterschiedlichen Positionen die Lage der mechanischen Achse des Femurs relativ zu diesem errechnet.

In der WO 98/40037 ist ein solches Verfahren beschrieben, wobei zur Bestimmung der mechanischen Achse des Femurs Markierelemente an der Hüfte und am Femur angebracht werden, deren Bewegungen beim Verschwenken des Femurs um das Hüftgelenk aufgenommen werden, die Lage des Hüftgelenks wird aus den Positionsdaten des Markierelementes an der Hüfte einerseits und am Femur andererseits bestimmt, und aus der Lage des Hüftgelenk und der in anderer Weise bestimmten Lage des Kniegelenkes läßt sich dann die mechanische Achse des Femurs bestimmen.

Dieses Verfahren arbeitet zuverlässig, hat aber den Nachteil, daß am Hüftknochen ein zusätzliches Markierelement angeordnet werden muß, und dies ist umständlich und für den Patienten unter Umständen mit zusätzlichen Schmerzen verbunden.

Es ist Aufgabe der Erfindung, ein gattungsgemäßes Verfahren so auszugestalten, daß eine einwandfreie Bestimmung der Lage der mechanischen Achse des Femurs auch möglich ist, wenn nur am Femur ein Markierelement angeordnet ist.

Diese Aufgabe wird bei einem Verfahren der eingangs beschriebenen Art erfindungsgemäß dadurch gelöst, daß man den Femur von einer Ausgangslage aus nur um einen maximalen Schwenkwinkel von 15° in unterschiedliche Richtungen verschwenkt und daß man aus den Positionsdaten der dabei von dem Markierelement überstrichenen Fläche und aus den anderweitig bestimmten Positionsdaten des Kniegelenks die mechanische Achse des Femurs berechnet.

Bei diesem Verfahren wird also der Femur relativ zum Beckenknochen, in dem der Femur drehbar gelagert ist, nur um einen sehr kleinen Schwenkwinkel verschwenkt, der maximal bei 15° liegt, vorzugsweise noch darunter, beispielsweise bei maximal 10° oder sogar noch weniger. Bei derart kleinen Schwenkwinkeln kann davon ausgegangen werden, daß das Becken durch diese Schwenkbewegung nicht nennenswert bewegt wird und auch ohne zusätzliche, eventuell schmerzhafte Fixierung seine Lage beibehält. Dadurch wird bei dieser sehr kleinen Schwenkbewegung des Femurs dieser um ein Hüftgelenk verschwenkt, das im Raum praktisch stationär angeordnet ist, und dies führt dazu, daß sich das Markierelement bei dieser Schwenkbewegung auf einer Kugelteilfläche bewegt, deren Mittelpunkt im wesentlichen durch das Hüftgelenk bestimmt wird. Aus den Positionsdaten dieser überstrichenen Kugelteilfläche läßt sich dann die Lage der mechanischen Achse des Femurs berechnen, ohne daß dazu eine Positionsüberwachung des Beckens notwendig ist. Es genügt also bei diesem Verfahren, die Bewegung des Femurs zu verfolgen und daher genügt es auch, wenn nur am Femur ein Markierelement festgelegt ist.

Bei einer bevorzugten Ausführungsform ist vorgesehen, daß man nur Positionsdaten für die Berechnung der mechanischen Achse verwendet, die einem Schwenkwinkel entsprechen, der unter einem bestimmten Grenzwinkel liegt, der kleiner ist als der vom Femur überstrichene maximale Schwenkwinkel. Insbesondere kann dieser Grenzwinkel zwischen 4° und 6° liegen.

Durch die Beschränkung auf solche Positionsdaten, die bei einer besonders kleinen Schwenkbewegung gewonnen werden, wird in erhöhtem Maße dafür Sorge getragen, daß bei der Verschwenkung das Becken nicht bewegt wird, das Hüftgelenk also stationär bleibt. Eine Verschwenkung in dieser Größenordnung führt zu einer relativ kleinen Schwenkfläche des Markierelementes, diese kann beispielsweise innerhalb eines Kreises mit einem Radius von 8 cm liegen.

Andererseits kann auch vorgesehen sein, daß man nur Positionsdaten für die Berechnung der mechanischen Achse verwendet, die einem Schwenkwinkel entsprechen, der über einem bestimmten Minimalwinkel liegt, der kleiner ist als der Grenzwinkel. Beispielsweise kann der Minimalwinkel über 3° liegen.

Bei einem solchen Verfahren werden also nur die Positionsdaten verwendet, die bei einer Auslenkung des Femurs aus der Anfangslage zwischen dem Minimalwinkel und dem Grenzwinkel liegen, also nur in einem schmalen Kreisringbereich, und dadurch läßt sich die Genauigkeit steigern, mit der die Positionsdaten der vom Markierelement überstrichenen Fläche bestimmt werden.

Insbesondere kann vorgesehen werden, daß man alle gespeicherten Positionsdaten unberücksichtigt läßt, wenn der tatsächliche Schwenkwinkel des Femurs relativ zu seiner Ausgangslage den maximalen Schwenkwinkel überschreitet. Mit anderen Worten wird eine solche Messung ungültig, wenn ein maximaler Schwenkwinkel überschritten wird, beispielsweise ein maximaler Schwenkwinkel von 15°, da dann die Gefahr besteht, daß durch den relativ großen Schwenkwinkel das Becken und damit das Hüftgelenk bewegt worden sind. Nur wenn ein solcher maximaler Schwenkwinkel bei der Schwenkbewegung nicht überschritten wird, werden die bei der Messung gespeicherten Positionsdaten für die anschließende Berechnung der mechanischen Achse des Femurs berücksichtigt. Wird der maximale Schwenkwinkel einmal überschritten, muß die gesamte Messung wiederholt werden.

Um von den gespeicherten Positionsdaten die Lage der mechanischen Achse des Femurs bestimmen zu können, wird vorzugsweise vorgesehen, daß man zur Berechnung der mechanischen Achse des Femurs aus der von dem Markierelement überstrichenen Kugelteilfläche den Mittelpunkt dieser Kugelteilfläche berechnet und die mechanische Achse durch die Verbindungslinie dieses Mittelpunktes mit dem Kniegelenk bestimmt.

Da die Kugelteilfläche sehr klein ist, ist die Genauigkeit eventuell etwas eingeschränkt, mit der die genaue Lage des Mittelpunktes der Kugelteilfläche bestimmt werden kann, insbesondere hinsichtlich des Abstandes dieses Mittelpunktes von der Kugelteilfläche.

Um hier eine Verbesserung vornehmen zu können, kann bei einer weiteren bevorzugten Ausführungsform der Erfindung vorgesehen sein, daß man zunächst einen virtuellen Mittelpunkt der Kugelfläche dadurch bestimmt, daß man aus den gespeicherten Positionsdaten der Kugelteilfläche angenähert eine Ebene und darauf eine durch das Kniegelenk verlaufende Senkrechte berechnet und den virtuellen Mittelpunkt in einem vorbestimmten Abstand von dieser Ebene auf der Senkrechten annimmt, und daß man dann unter Verwendung der Positionsdaten des virtuellen Mittelpunktes und der Positionsdaten der Kugelteilfläche die mechanische Achse des Femurs berechnet. Der vorbestimmte Abstand hängt dabei natürlich von der Positionierung des Markierelementes am Femur ab, dieser vorbestimmte Abstand entspricht etwa dem Abstand des Markierelementes vom Hüftgelenk, der abschätzbar ist und beispielsweise 40 cm betragen kann, diese Größe beeinflußt die Genauigkeit der Berechnungsmethode nur relativ geringfügig.

Es ist weiterhin vorteilhaft, wenn man die Positionsdaten der Kugelteilfläche unter Verwendung der Positionsdaten des virtuellen Mittelpunktes auf einen einheitlichen Schwenkwinkel relativ zur Ausgangslage umrechnet, so daß man daraus einen gemeinsamen Kreis definierende, korrigierte Positionsdaten erhält, und daß man die Mittelsenkrechte dieses Kreises als mechanische Achse des Femurs berechnet. Es werden also unter Verwendung der Positionsdaten des virtuellen Mittelpunktes alle gewonnen Positionsdaten so umgerechnet, d. h. virtuell um den virtuellen Mittelpunkt verschwenkt, daß die Positionsdaten einem Schwenkwinkel entsprechen, der durch den gemeinsamen Kreis hindurchgeht. Es hat sich gezeigt, daß bei dieser Berechnungsmethode auch ausgehend von einer relativ kleinen Kugelteilfläche die mechanische Achse des Femurs mit großer Genauigkeit bestimmt werden kann.

Bei einer weiteren bevorzugten Ausführungsform der Erfindung ist vorgesehen, daß man die Zahl und die Verteilung der gemessenen Positionsdaten im gesamten Schwenkbereich bestimmt und nach Erreichen einer vorgegebenen Zahl und Verteilung die Aufnahme von weiteren Positionsdaten abbricht. Damit ist sichergestellt, daß man die Messung auf jeden Fall solange weiterführt, bis im gesamten Schwenkbereich eine genügende Anzahl von gemessenen Positionsdaten zur Verfügung steht, um die räumliche Anordnung der von dem Markierelement überstrichenen kugelteilfläche mit genügender Genauigkeit bestimmen zu können.

Günstig ist es auch, wenn man die gespeicherten Positionsdaten entsprechend ihrer räumlichen Verteilung im Schwenkbereich dieser entsprechend bildlich darstellt, so daß erkennbar ist, für welchen Teil des möglichen Schwenkbereiches welche Anzahl von Positionsdaten gespeichert worden ist. Der Operateur kann an dieser bildlichen Darstellung sofort erkennen, in welchem Bereich des Schwenkbereiches noch zusätzliche Positionsdaten bestimmt werden müssen, d. h. er kann den Femur in diesen noch nicht ausreichend vermessenen Schwenkbereich verschwenken.

Insbesondere kann vorgesehen sein, daß man in einer bildlich dargestellten Fläche Teilbereiche dieser Fläche markiert, wenn in einem diesem Teilbereich entsprechenden Teil des Schwenkbereichs des Femurs eine bestimmte Anzahl von Positionsdaten aufgenommen ist. Insbesondere kann diese Fläche eine in Segmente unterteilte Ringfläche sein. Der Operateur kann damit unmittelbar an dieser Darstellung erkennen, ob in einem bestimmten Bereich ausreichend Positionsdaten vorliegen oder nicht, beispielsweise kann dies durch einen Umschlag der Farbe eines Teilbereichs der Fläche erfolgen.

Die Erfindung betrifft weiterhin eine Vorrichtung zur Durchführung dieses Verfahrens mit einem Navigationssystem zur Bestimmung von Positionsdaten eines am Femur festgelegten Markierelementes und mit einer Datenverarbeitungsanlage zur Berechnung der Lage der mechanischen Achse des Femurs aus diesen Positionsdaten.

Der Erfindung liegt dementsprechend auch die Aufgabe zugrunde, eine gattungsgemäße Vorrichtung so auszubilden, daß mit ihr ohne Verwendung eines zweiten Markier-etementes im Beckenbereich eine exakte Bestimmung der mechanischen Achse des Femurs möglich ist, ohne daß dazu eine spezielle Festlegung des Beckens des Patienten notwendig wird.

Diese Aufgabe wird bei einer Vorrichtung der eingangs beschriebenen Art erfindungsgemäß dadurch gelöst, daß die Datenverarbeitungsanlage aus den Positionsdaten zur Berechnung der Lage der mechanischen Achse des Femurs solche auswählt, die einem Schinrenkwinkel entsprechen, der unter einem bestimmten Grenzwinkel liegt, der kleiner ist als der vom Femur überstrichene maximale Schwenkwinkel. Diese Beschränkung auf Positionsdaten, die bei Bewegung um kleine Schwenkwinkel gewonnen worden sind, stellt sicher, daß bei diesen kleinen Schwenkbewegungen das Becken und damit das Hüftgelenk stationär geblieben sind, so daß sich für die Bewegung des Markierelementes eine Bewegung auf einer Kugelteilfläche ergibt, deren Positionsdaten durch die Verschwenkung bestimmt und zur weiteren Berechnung verwendet werden können.

Weitere vorteilhafte Ausgestaltungen einer solchen Vorrichtung ergeben sich aus den Patentansprüchen.

Die nachfolgende Beschreibung einer bevorzugten Ausführungsform der Erfindung dient im Zusammenhang mit der Zeichnung der näheren Erläuterung. Es zeigen:
- Figur 1:: eine schematische Ansicht einer Vorrichtung zur Bestimmung der mechanischen Achse eines Femurs;
- Figur 2:: ein in den Femur eingesetztes Markierelement;
- Figur 3:: eine schematische Darstellung der vom Femur und damit vom Markierelement vorgenommenen Schwenkbewegung;
- Figur 4a:: eine schematische Darstellung eines Bildschirmes zur Überwachung der Aufnahme von Positionsdaten des Markierelementes bei der Verschwenkbewegung eines Femurs vor Beginn dieser Aufnahme und
- Figur 4b:: eine Ansicht ähnlich Figur 4a bei Beendigung der Aufnahme.

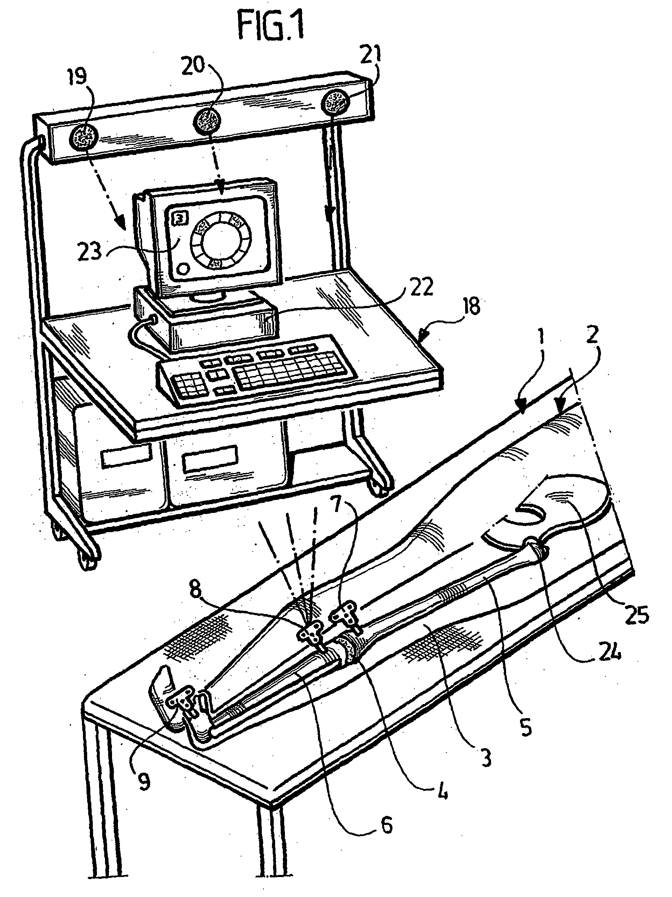

In Figur 1 ist ein auf einem Operationstisch 1 liegender Patient 2 schematisch dargestellt, bei dem in einem Bein 3 das Kniegelenk 4 durch eine Endoprothese ersetzt werden soll.

Zur Vorbereitung dieser Operation ist es notwendig, die Orientierung der zu verwendenden Prothesenteile relativ zu den Knochen zu bestimmen, also relativ zum Femur oder Oberschenkelknochen 5 und gegebenenfalls auch zum Unterschenkelknochen 6.

Zu diesem Zweck wird in den Femur 5 in der Nähe des Kniegelenks 4 ein Markierelement 7 eingesetzt, beispielsweise durch Einschrauben, außerdem noch entsprechende Markierelemente 8, 9 in den Unterschenkelknochen 6, die jedoch für das hier interessierende Verfahren nicht von Bedeutung sind.

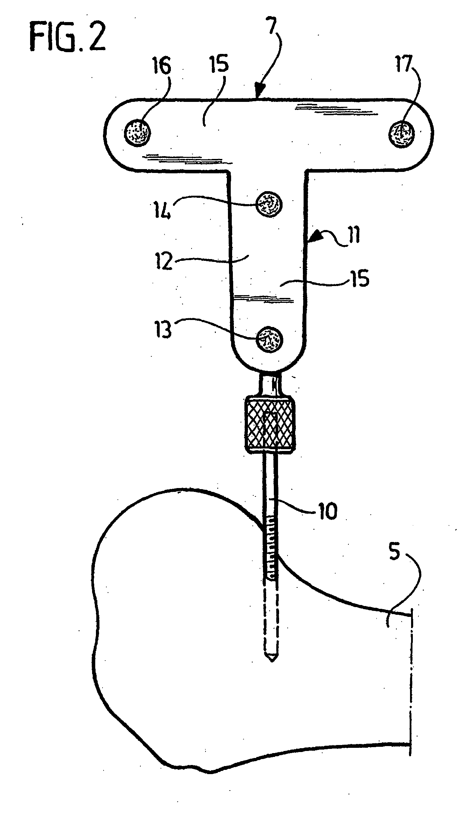

In Figur 2 ist ein derartiges Markierelement 7 dargestellt, es umfaßt einen in den Femur einschraubbaren Fuß 10 in Form einer Knochenschraube und einen T-förmigen Aufsetzkörper 11, der an seinem parallel zum Fuß 10 verlaufenden Steg 12 im Abstand zueinander zwei Strahlungssender 13, 14 und an seinem an den Steg 12 anschließenden Quersteg 15 ebenfalls zwei Strahlungssender 16, 17 trägt. Diese Strahlungssender können beispielsweise Ultrarotdioden sein oder Ultraschallsender. Der Aufsetzkörper 11 kann lösbar auf den Fuß 10 aufgesetzt sein, allerdings nur in einer ganz bestimmten Position, so daß auch nach der Abnahme und nach dem Wiederaufsetzen eines solchen Aufsetzkörpers 11 die Strahlungssender 13, 14, 16, 17 relativ zum Knochen exakt dieselbe Position einnehmen wie vor dem Abnehmen.

An einer Konsole 18 sind im Abstand zueinander drei Empfangseinrichtungen 19, 20, 21 angeordnet, die die Strahlung empfangen, die von den Strahlungssendern 13, 14, 16, 17 ausgesandt werden. Beim Empfang von Strahlung erzeugen die Empfangseinrichtungen'elektrische Signale, die einer Datenverarbeitungsanlage 22 zugeführt werden. Aufgrund der unterschiedlichen Orientierung von Markierelementen und Empfangseinrichtungen ergeben sich Laufzeitunterschiede zwischen Aussenden und Empfangen der Strahlung, und aufgrund dieser Laufzeitunterschiede kann die Datenverarbeitungsanlage 22 bei dem Markierelement 7 dessen Lage im Raum vollständig bestimmen und diese Lagedaten speichern. Es ist dadurch möglich, in der Datenverarbeitungsanlage 22 Datensätze zu erzeugen, die der Lage des Markierelementes 7 und damit des fest mit ihm verbundenen Femurs 5 zu bestimmten Zeiten entsprechen.

Die Empfangseinrichtungen 19, 20, 21 können in unterschiedlicher Weise ausgebildet sein, sie können, wie beschrieben, die Orientierung des Markierelementes durch Laufzeitunterschiede feststellen, grundsätzlich möglich wäre auch die Bestimmung der Orientierung durch geometrische Messung der Strahlenrichtung von Strahlen, die von den Strohlungssendern 13, 14, 16, 17 ausgesandt wird.

Bei anderen Ausgestaltungen können auch Markierelemente verwendet werden, die keine Strahtungssender aufweisen, sondern Reflexionsflächen, an denen von der Empfangseinrichtung ausgesandte Strahlung reflektiert wird. Diese Reflexionsflächen können beispielsweise Kugelform haben.

Wesentlich ist lediglich, daß es aufgrund der Verwendung von mehreren Empfangseinrichtungen und mehreren Sendern oder Reflexionsflächen an dem Markierelement möglich ist, die Lage des Markierelementes im Raum eindeutig zu bestimmen. Eine solche Anordnung wird allgemein als Navigationssystem bezeichnet.

Die Datenverarbeitungsanlage 22 ist mit einem Bildschirm 23 versehen, auf dem in Abhängigkeit von aufgenommenen Positionsdaten Informationen für den Benutzer angezeigt werden.

Zur Bestimmung der mechanischen Achse des Femurs 5 wird der Femur 5 von einer beliebigen Ausgangslage aus um einen Drehpunkt verschwenkt, der durch das Hüftgelenk 24 gebildet wird, welches den Femur 5 am Beckenknochen 25 verschwenkbar lagert. Bei dem hier beschriebenen Verfahren verschwenkt der Operateur den Femur 5 ausgehend von dieser Ausgangslage nach allen Richtungen um einen relativ kleinen Schwenkwinkel, der beispielsweise in der Größenordnung von 5° oder knapp darüber liegt, auf keinen Fall jedoch einen maximalen Schwenkwinkel überschreitet, der beispielsweise bei 15° liegen kann. Es wird also nur eine sehr geringe Schwenkbewegung vorgenommen, und dies führt dazu, daß bei dieser geringen Schwenkbewegung der Beckenknochen 25 des Patienten stationär bleibt, ohne daß dazu besondere Fixiermaßnahmen notwendig sind.

Beim Verschwenken des Femurs 5 in dem beschriebenen Schwenkbereich bewegt sich das Markierelement 7 auf einer Kugelteilfläche, deren Mittelpunkt im Hüftgelenk 24 angeordnet ist. Die jeweilige Lage des Markierelementes 7 wird bei der gesamten Verschwenkbewegung durch das Navigationssystem bestim mt und entsprechende Datensätze werden in der Datenverarbeitungsanlage 22 gespeichert. Diese Datensätze geben die Lage des Markierelementes 7 zu verschiedenen Zeiten während der Verschwenkbewegung an. Da der Operateur den Femur während der Verschwenkung ausgehend von der Ausgangslage in alle Richtungen verschwenkt, verteilen sich somit die Positionen des Markierelementes während der Dauer der Verschwenkung über die gesamte Kugelteilfläche, die einen Verschwenkkegel mit einem Öffnungswinkel von maximal 15° an der Basis begrenzt. Die Spitze dieses Verschwenkkegels liegt im Hüftgelenk 24.

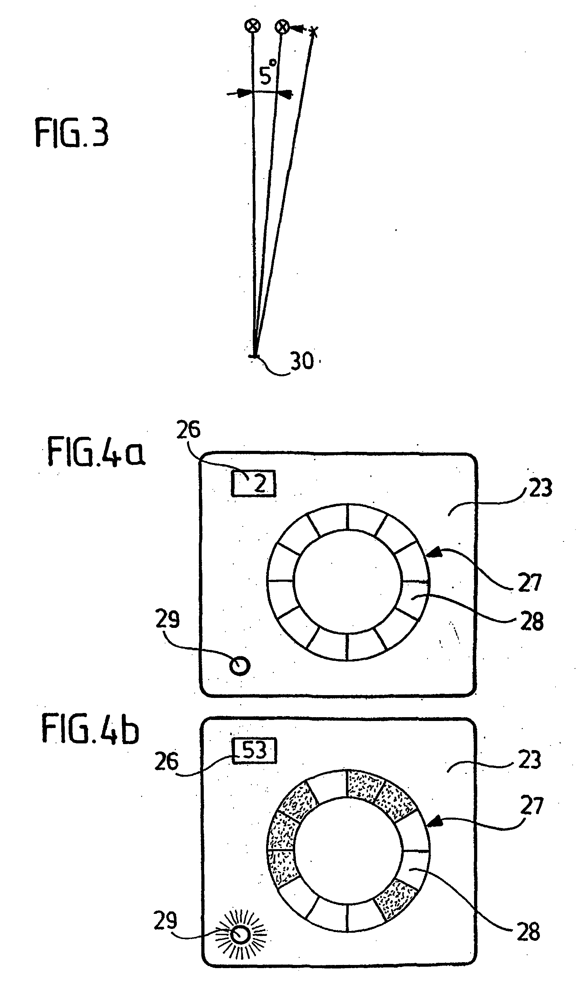

Die Zahl der bei dieser Verschwenkbewegung bestimmten Positionsdaten wird in einem speziellen Fenster 26 des Bildschirmes 23 angezeigt, auf diesem Bildschirm ist außerdem ein Kreisring 27 dargestellt, der in eine Anzahl von Einzelsegmenten 28 unterteilt ist. Während der Aufnahme der Positionsdaten werden nicht nur die aufgenommenen Positionsdaten insgesamt gezählt, sondern es wird auch für jede Teilfläche des Verschwenkbereichs bestimmt, wie viele Positionsdaten in dieser Teilfläche bestimmt worden sind. Jede dieser Teilflächen ist einem Einzelsegment des dargestellten Kreisringes 27 zugeordnet, und sobald in einer bestimmten Teilfläche genügend Positionsdaten gesammelt worden sind, wird das entsprechende Einzelsegment 28 markiert, beispielsweise durch einen Farbumschlag. In der Darstellung der Figur 4b sind Einzelsegmente 28 dunkel markiert, die Teilflächen zugeordnet sind, in denen bereits genügend Positionsdaten gesammelt sind, bei den hellen Einzelsegmenten 28 dagegen hat die Zahl der aufgenommenen Positionsdaten noch nicht eine bestimmte Größe erreicht. Der Operateur kann daraus ohne weiteres ablesen, in welche Richtung noch zusätzliche Verschwenkbewegungen notwendig sind, um auch in diesem Bereich die notwendige Zahl von Messungen vorzunehmen.

Die Datenverarbeitungsanlage 22 überwacht, daß der Verschwenkwinkel nicht über einen maximalen Schwenkwinkel hinaus vergrößert wird, beispielsweise kann dieser maximale Schwenkwinkel bei 15° liegen. Wenn der Operateur diesen Schwenkwinkel überschreitet, wird automatisch ein Signal angezeigt, beispielsweise ein Leuchtsignal 29 auf dem Bildschirm, und alle bis dahin bestimmten Positionsdaten werden verworfen. Der Meßvorgang muß dann wiederholt werden, da nicht auszuschließen ist, daß beim Überschreiten des maximalen Schwenkwinkels der Beckenknochen 25 bewegt worden ist, so daß das Hüftgelenk 24 nicht stationär geblieben ist.

Wenn ein Meßvorgang in dieser Weise ohne Abbruch, d. h. ohne Überschreiten des maximalen Schwenkwinkels, beendet worden ist, steht ein Datensatz mit einer größeren Anzahl von Positionsdaten des Markierelementes 7 zur Verfügung. Die Datenverarbeitungsanlage 22 wählt aus diesen Datensätzen solche aus, bei denen der Schwenkwinkel über einem minimalen Schwenkwinkel liegt, beispielsweise in der Größenordnung von 3°, und unterhalb eines maximalen Grenzwinkels, beispielsweise in der Größenordnung von 6°. Es werden also nur Positionsdaten für die Weiterverarbeitung berücksichtigt, die Schwenkwinkeln zwischen dem minimalen Schwenkwinkel und dem Grenzwinkel entsprechen, die also im hier dargelegten Beispiel zwischen 3° und 6° liegen.

Grundsätzlich wäre es möglich, aus den Positionsdatensätzen, die eine Kugelteilfläche beschreiben, direkt den Mittelpunkt der Kugelteilfläche zu berechnen, dieser Mittelpunkt gibt dann die Lage des Hüftgelenkes 24 an. Verbindet man die Position dieses Mittelpunktes mit der Position des Kniegelenkes 4, die auf andere Weise ermittelt werden kann, beispielsweise durch Tasten, dann ergibt diese Verbindungslinie die mechanische Achse des Femurs, die für die Orientierung von Operationswerkzeugen benutzt werden kann.

In dieser Weise kann grundsätzlich vorgegangen werden, da die Teilkugelfläche, die das Markierelement 7 überschreibt, jedoch sehr klein ist, können sich bei dieser direkten Bestimmung des Mittelpunkts der Kugelteilfläche Beschränkungen bei der Genauigkeit ergeben.

Eine Verbesserung läßt sich erzielen, wenn aus den Positionsdaten durch ein mathematisches Näherungsverfahren zunächst eine Ebene berechnet wird, die angenähert durch die Positionen des Markierelementes 7 beim Verschwenkvorgang hindurchgeht, und wenn daraus eine Linie berechnet wird, die senkrecht auf dieser Ebene steht und durch das Kniegelenk hindurchgeht. Entsprechend der jeweiligen Anordnung des Markierelementes 7 am Femur 5 kann dann ein virtueller Mittelpunkt bestimmt werden, der auf dieser Linie liegt und von der berechneten Ebene einen Abstand einnimmt, der in etwa dem tatsächlichen Abstand des Markierelementes 7 vom Hüftgelenk 24 entspricht, diese zuletzt genannte Größe ist nicht sehr kritisch und kann relativ grob in die Bestimmung des virtuellen Mittelpunktes eingehen.

Dieser virtuelle Mittelpunkt wird in der Nähe des tatsächlichen Hüftgelenkes 24 liegen und wird für einen nächsten Rechenschritt eingesetzt. Bei diesem Rechenschritt werden alle Positionsdaten, die unterschiedlichen Schwenkwinkeln zugrundeliegen, umgerechnet in Positionsdaten, die alle dehseiben Schwenkwinkel aufweisen. Mit anderen Worten werden die ursprünglich aufgefundenen Positionsdaten um den virtuellen Mittelpunkt verschwenkt, bis der Schwenkwinkel für alle Positionsdaten gleich ist, es werden also alle Positionsdaten auf einen gemeinsamen Kreis geschwenkt, beispielsweise mit einem Öffnungswinkel von 5°. Dies ist in Figur 3 angedeutet, dort wird ein Satz von Positionsdaten für einen Schwenkwinkel, der größer als 5° ist, um den virtuellen Mittelpunkt 30 rechnerisch verschwenkt, bis er einen Schwenkwinkel von 5° erreicht hat. Auf diese Weise können alle gemessenen Positionsdaten zur Festlegung eines Kreises verwendet werden, und die mechanische Achse des Femurs läßt sich nun aus den geometrischen Daten dieses Kreises sehr einfach dadurch bestimmen, daß die Mittelachse dieses Kreises bestimmt wird.

Durch das beschriebene Verfahren läßt sich die Genauigkeit der Lagebestimmung für die mechanische Achse des Femurs verbessern, es wird dadurch möglich, selbst bei einer sehr kleinen Kugelteilfläche, d.h. bei sehr kleinen Verschwenkwinkeln, mit der erforderlichen Genauigkeit die Lage der mechanischen Achse des Femurs zu berechnen.

## Patentansprüche

1. Verfahren zur Bestimmung der mechanischen Achse eines Femurs, bei dem man den Femur um das Hüftgelenk bewegt, die Bewegung des Femurs mittels eines Markierelementes am Femur über ein Navigationssystem verfolgt, daraus gewonnene Positionsdaten des Femurs speichert und aus den unterschiedlichen Positionsdaten des Femurs in unterschiedlichen Positionen die Lage der mechanischen Achse des Femurs relativ zu diesem errechnet, **dadurch gekennzeichnet, daß** man den Femur von einer Ausgangslage aus nur um einen maximalen Schwenkwinkel von 15° in unterschiedliche Richtungen verschwenkt und daß man aus den Positionsdaten der dabei von dem Markierungselement überstrichenen Fläche und aus den anderweitig bestimmten Positionsdaten des Kniegelenkes die mechanische Achse des Femurs berechnet.

2. Verfahren nach Anspruch 1, **dadurch gekennzeichnet, daß** man den Femur nur innerhalb eines maximalen Schwenkwinkels von maximal 10° verschwenkt.

3. Verfahren nach Anspruch 1 oder 2, **dadurch gekennzeichnet, daß** man nur Positionsdaten für die Berechnung der mechanischen Achse verwendet, die einem Schwenkwinkel entsprechen, der unter einem bestimmten Grenzwinkel liegt, der kleiner ist als der vom Femur überstrichene maximale Schwenkwinkel.

4. Verfahren nach Anspruch 3, **dadurch gekennzeichnet, daß** der Grenzwinkel zwischen 4° und 6° liegt.

5. Verfahren nach einem der Ansprüche 3 oder 4, **dadurch gekennzeichnet, daß** man nur Positionsdaten für die Berechnung der mechanischen Achse verwendet, die einem Schwenkwinkel entsprechen, der über einem bestimmten Minimalwinkel liegt, der kleiner ist als der Grenzwinkel.

6. Verfahren nach Anspruch 5, **dadurch gekennzeichnet, daß** der Minimalwinkel über 3° liegt.

7. Verfahren nach einem der voranstehenden Ansprüche, **dadurch gekennzeichnet, daß** man alle gespeicherten Positionsdaten unberücksichtigt läßt, wenn der tatsächliche Schwenkwinkel des Femurs relativ zu seiner Ausgangslage den maximalen Schwenkwinkel überschreitet.

8. Verfahren nach einem der voranstehenden Ansprüche, **dadurch gekennzeichnet, daß** man zur Berechnung der mechanischen Achse des Femurs aus der von dem Markierelement überstrichenen Kugelteilfläche den Mittelpunkt dieser Kugelteilfläche berechnet und die mechanische Achse durch die Verbindungslinie dieses Mittelpunktes mit dem Kniegelenk bestimmt.

9. Verfahren nach Anspruch 8, **dadurch gekennzeichnet, daß** man zunächst einen virtuellen Mittelpunkt der Kugelteilfläche dadurch bestimmt, daß man aus den gespeicherten Positionsdaten der Kugelteilfläche angenähert eine Ebene und darauf eine durch das Kniegelenk verlaufende Senkrechte berechnet und den virtuellen Mittelpunkt in einem vorbestimmten Abstand von dieser Ebene auf der Senkrechten annimmt, und daß man dann unter Verwendung der Positionsdaten des virtuellen Mittelpunktes und der Positionsdaten der Kugelteilfläche die mechanische Achse des Femurs berechnet.

10. Verfahren nach Anspruch 9, **dadurch gekennzeichnet, daß** man die Positionsdaten der Kugelteilfläche unter Verwendung der Positionsdaten des virtuellen Mittelpunktes auf einen einheitlichen Schwenkwinkel relativ zur Ausgangslage umrechnet, so daß man daraus einen gemeinsamen Kreis definierende, korrigierte Positionsdaten erhält, und daß man die Mittelsenkrechte dieses Kreises als mechanische Achse des Femurs berechnet.

11. Verfahren nach einem der voranstehenden Ansprüche, **dadurch gekennzeichnet, daß** man die Zahl und die Verteilung der gemessenen Positionsdaten im gesamten Schwenkbereich bestimmt und nach Erreichen einer vorgegebenen Zahl und Verteilung die Aufnahme von weiteren Positionsdaten abbricht.

12. Verfahren nach einem der voranstehenden Ansprüche, **dadurch gekennzeichnet, daß** man die gespeicherten Positionsdaten entsprechend ihrer räumlichen Verteilung im Schwenkbereich dieser entsprechend bildlich darstellt, so daß erkennbar ist, für welchen Teil des möglichen Schwenkbereiches weiche Anzahl von Positionsdaten gespeichert worden ist.

13. Verfahren nach Anspruch 12, **dadurch gekennzeichnet, daß** man in einer bildlich dargestellten Fläche Teilbereiche dieser Fläche markiert, wenn in einem diesem Teilbereich entsprechenden Teil des Schwenkbereichs des Femurs eine bestimmte Anzahl von Positionsdaten aufgenommen ist.

14. Verfahren nach Anspruch 13, **dadurch gekennzeichnet, daß** die Fläche eine in Segmente unterteilte Ringfläche ist.

15. Vorrichtung zur Durchführung des Verfahrens der Ansprüche 1 bis 14, mit einem Navigationssystem zur Bestimmung von Positionsdaten eines am Femur festgelegten Markierelementes und mit einer Datenverarbeitungsanlage zur Berechnung der Lage der mechanischen Achse des Femurs aus diesen Positionsdaten, **dadurch gekennzeichnet, daß** die Datenverarbeitungsanlage (22) aus den Positionsdaten zur Berechnung der Lage der mechanischen Achse des Femurs (5) solche auswählt, die unter einem bestimmten Grenzwinkel liegen, der kleiner ist als der vom Femur (5) überstrichene maximale Schwenkwinkel.

16. Vorrichtung nach Anspruch 15, **dadurch gekennzeichnet, daß** der Grenzwinkel zwischen 4° und 6° liegt.

17. Vorrichtung nach Anspruch 15 oder 16, **dadurch gekennzeichnet, daß** die Datenverarbeitungsanlage (22) aus den Positionsdaten zur Berechnung der Lage der mechanischen Achse des Femurs (5) solche auswählt, die über einem bestimmten Minimalwinkel liegen, der kleiner ist als der Grenzwinkel.

18. Vorrichtung nach Anspruch 17, **dadurch gekennzeichnet, daß** der Minimalwinkel über 3° liegt.

19. Vorrichtung nach einem der Ansprüche 15 bis 18, **dadurch gekennzeichnet, daß** die Datenverarbeitungsanlage (22) alle gespeicherten Positionsdaten unberücksichtigt läßt, wenn der tatsächliche Schwenkwinkel des Femurs (5) relativ zu seiner Ausgangslage den maximalen Schwenkwinkel überschreitet.

20. Vorrichtung nach einem der Ansprüche 15 bis 19, **dadurch gekennzeichnet, daß** die Datenverarbeitungsanlage (22) zur Berechnung der mechanischen Achse des Femurs (5) aus der von dem Markierelement (7) überstrichenen Kugelteilfläche den Mittelpunkt dieser Kugelteilfläche berechnet und die mechanische Achse durch die Verbindungslinie dieses Mittelpunktes mit dem Kniegelenk (4) bestimmt.

21. Vorrichtung nach Anspruch 20, **dadurch gekennzeichnet, daß** die Datenverarbeitungsanlage (22) zunächst einen virtuellen Mittelpunkt (30) der Kugelteilfläche dadurch bestimmt, daß sie aus den gespeicherten Positionsdaten der Kugelteilfläche angenähert eine Ebene und darauf eine durch das Kniegelenk (4) verlaufende Senkrechte berechnet und den virtuellen Mittelpunkt (30) in einem vorbestimmten Abstand von dieser Ebene auf der Senkrechten annimmt, und daß die Datenverarbeitungsanlage (22) dann unter Verwendung der Positionsdaten des virtuellen Mittelpunktes (30) und der Positionsdaten der Kugelteilfläche die mechanische Achse des Femurs (5) berechnet.

22. Vorrichtung nach Anspruch 21, **dadurch gekennzeichnet, daß** die Datenverarbeitungsanlage (22) die Positionsdaten der Kugelteilfläche unter Verwendung der Positionsdaten des virtuellen Mittelpunktes (30) auf einen einheitlichen Schwenkwinkel relativ zur Ausgangslage umrechnet, so daß man daraus einen gemeinsamen Kreis definierende, korrigierte Positionsdaten erhält, und daß sie die Mittelsenkrechte dieses Kreises als mechanische Achse des Femurs (5) berechnet.

23. Vorrichtung nach einem der Ansprüche 15 bis 22, **dadurch gekennzeichnet, daß** die Datenverarbeitungsanlage (22) die Zahl und die Verteilung der gemessenen Positionsdaten im gesamten Schwenkbereich bestimmt und nach Erreichen einer vorgegebenen Zahl und Verteilung die Aufnahme von weiteren Positionsdaten abbricht.

24. Vorrichtung nach einem der Ansprüche 21 bis 23, **dadurch gekennzeichnet, daß** die Datenverarbeitungsanlage (22) die gespeicherten Positionsdaten entsprechend ihrer räumlichen Verteilung im Schwenkbereich dieser entsprechend bildlich darstellt, so daß erkennbar ist, für welchen Teil des möglichen Schwenkbereichs welche Anzahl von Positionsdaten gespeichert Worden ist.

25. Vorrichtung nach Anspruch 24, **dadurch gekennzeichnet, daß** die Datenverarbeitungsanlage (22) in einer bildlich dargestellten Fläche (27) Teilbereiche (28) dieser Fläche (27) markiert, wenn in einem diesem Teilbereich (28) entsprechenden Teil des Schwenkbereiches des Femurs (5) eine bestimmte Anzahl von Positionsdaten aufgenommen ist.

26. Vorrichtung nach Anspruch 25, **dadurch gekennzeichnet, daß** die Fläche (27) eine in Segmente (28) unterteilte Ringfläche ist.

## Claims

1. A method for determining the mechanical axis of a femur, in which the femur is moved about the hip joint, the movement of the femur is monitored via a navigation system with the use of a marker on the femur, femur-position data obtained therefrom are stored and, from the various femur-position data in various positions, the orientation of the mechanical axis of the femur relative to the femur is calculated, **characterised in that**, from a starting position, the femur is swivelled only through a maximum swivel angle of 15° in various directions, and **in that** the mechanical axis of the femur is calculated from the position data for the area swept by the marker during this operation and from otherwise-determined knee-joint position data.

2. A method according to Claim 1, **characterised in that** the femur is swivelled only within a maximum swivel angle of maximally 10°.

3. A method according to Claim 1 or 2, **characterised in that** the mechanical axis is calculated only with the use of position data corresponding to a swivel angle smaller than a defined limit angle which is smaller than the maximum swivel angle swept by the femur.

4. A method according to Claim 3, **characterised in that** the limit angle is between 4° and 6°.

5. A method according to one of Claims 3 or 4, **characterised in that** the mechanical axis is calculated only with the use of position data corresponding to a swivel angle above a defined minimum angle which is smaller than the limit angle.

6. A method according to Claim 5, **characterised in that** the minimum angle is greater than 3°.

7. A method according to one of the preceding claims, **characterised in that** all stored position data are ignored if the actual swivel angle of the femur relative to its starting position exceeds the maximum swivel angle.

8. A method according to one of the preceding claims, **characterised in that**, for the purpose of calculating the mechanical axis of the femur from the partial-sphere surface swept by the marker, the centre point of the said partial-sphere surface is calculated and the mechanical axis is determined from the line connecting this centre point to the knee joint.

9. A method according to Claim 8, **characterised in that** a virtual centre point of the partial-sphere surface is first of all determined by calculating a plane and a line through the knee joint perpendicular thereto by approximation from the stored position data for the partial-sphere surface, and taking the virtual centre point at a predefined distance from this plane on the perpendicular, and **in that** the mechanical axis of the femur is calculated using the position data for the virtual centre point and the position data for the partial-sphere surface.

10. A method according to Claim 9, **characterised in that**, using the position data for the virtual centre point, the position data for the partial-sphere surface are converted to a uniform swivel angle relative to the starting orientation, with the result that corrected position data defining a common circle are obtained therefrom, and **in that** the mean perpendicular of this circle is calculated as the mechanical axis of the femur.

11. A method according to one of the preceding claims, **characterised in that** the number and the distribution of the measured position data within the whole swivel range are determined and the acquisition of further position data is terminated once a preselected number and distribution has been reached.

12. A method according to one of the preceding claims, **characterised in that** the stored position data are graphically displayed in correspondence with their spatial distribution within the swivel range, so that it can be perceived which population of position data has been stored for which part of the possible swivel range.

13. A method according to Claim 12, **characterised in that** when a defined number of position data have been acquired within a part of the swivel range of the femur corresponding to a particular partial range, partial regions of a graphically displayed area are marked on the said area.

14. A method according to Claim 13, **characterised in that** the area is an annular area subdivided into segments.

15. A device for performing the method according to Claims 1 to 14, having a navigation system for the determination of position data of a marker attached to the femur, and having a data-processing system for calculating the orientation of the mechanical axis of the femur from the said position data, **characterised in that**, from the position data for calculating the orientation of the mechanical axis of the femur (5), the data-processing system (22) selects ones below a defined limit angle which is smaller than the maximum swivel angle swept by the femur (5).

16. A device according to Claim 15, **characterised in that** the limit angle is between 4° and 6°.

17. A device according to Claim 15 or 16, **characterised in that**, from the position data for calculating the orientation of the mechanical axis of the femur (5), the data-processing system (22) selects ones above a defined minimum angle which is smaller than the limit angle.

18. A device according to Claim 17, **characterised in that** the minimum angle is larger than 3°.

19. A device according to one of Claims 15 to 18, **characterised in that** the data-processing system (22) ignores all stored position data if the actual swivel angle of the femur (5) relative to its starting position exceeds the maximum swivel angle.

20. A device according to one of Claims 15 to 19, **characterised in that**, for the purpose of calculating the mechanical axis of the femur (5) from the partial-sphere surface swept by the marker (7), the data-processing system (22) calculates the centre point of the said partial-sphere surface and determines the mechanical axis from the line connecting this centre point to the knee joint (4).

21. A device according to Claim 20, **characterised in that** the data-processing system (22) first of all determines a virtual centre point (30) of the partial-sphere surface by calculating a plane and a line through the knee joint (4) perpendicular thereto by approximation from the stored position data of the partial-sphere surface and taking the virtual centre point (30) at a predetermined distance from this plane on the perpendicular, and **in that** the data-processing system (22) then calculates the mechanical axis of the femur using the position data of the virtual centre point (30) and the position data of the partial-sphere surface.

22. A device according to Claim 21, **characterised in that** the data-processing system (22) converts the position data for the partial-sphere surface to a uniform swivel angle relative to the starting orientation, with the result that corrected position data defining a common circle are obtained therefrom, and **in that** it calculates the mean perpendicular of this circle as the mechanical axis of the femur.

23. A device according to one of Claims 15 to 22, **characterised in that** the data-processing system (22) and determines the number and the distribution of the measured position data within the whole swivel range and terminates the acquisition of further position data once a preselected number and distribution has been reached.

24. A device according to one of Claims 21 to 23, **characterised in that** the data-processing system (22) graphically displays the stored position data in correspondence with their spatial distribution within the swivel range, so that it can be perceived which population of position data has been stored for which part of the possible swivel range.

25. A device according to Claim 24, **characterised in that**, when a defined number of position data have been acquired within a part of the swivel range of the femur (5) corresponding to a particular partial range (28), the data-processing system (22) marks partial regions (28) of a graphically displayed area (27) on the said area (27).

26. A device according to Claim 25, **characterised in that** the area (27) is an annular area subdivided into segments (28).

## Revendications

1. Procédé pour déterminer l'axe mécanique d'un fémur, selon lequel on déplace le fémur autour de l'articulation de la hanche, on suit, par l'intermédiaire d'un système de navigation, le mouvement du fémur au moyen d'un élément de marquage sur le fémur, on mémorise des données de position du fémur ainsi récoltées, et on calcule, à partir des différentes données de position du fémur, la position de l'axe mécanique du fémur relativement à celui-ci, pour différentes positions,
**caractérisé en ce que** l'on ne fait pivoter le fémur, à partir d'une position initiale, que d'un angle de pivotement maximal de 15° dans différentes directions, et **en ce que** l'on calcule l'axe mécanique du fémur à partir des données de position de la surface balayée à cette occasion par l'élément de marquage, et à partir des données de position de l'articulation du genou, qui ont été déterminées autrement.

2. Procédé selon la revendication 1, **caractérisé en ce que** l'on ne fait pivoter le fémur qu'à l'intérieur d'un angle de pivotement maximal d'au maximum 10°.

3. Procédé selon la revendication 1 ou 2, **caractérisé en ce que** pour le calcul de l'axe mécanique, on n'utilise que des données de position qui correspondent à un angle de pivotement situé en-dessous d'un angle limite déterminé, qui est inférieur à l'angle de pivotement maximal balayé par le fémur.

4. Procédé selon la revendication 3, **caractérisé en ce que** l'angle limite se situe entre 4° et 6°.

5. Procédé selon l'une des revendications 3 ou 4, **caractérisé en ce que** pour le calcul de l'axe mécanique, on n'utilise que des données de position qui correspondent à un angle de pivotement situé au-dessus d'un angle minimal déterminé, qui est inférieur à l'angle limite.

6. Procédé selon la revendication 5, **caractérisé en ce que** l'angle minimal se situe au-dessus de 3°.

7. Procédé selon l'une des revendications précédentes, **caractérisé en ce que** l'on ne prend en considération aucune de toutes les données de position mémorisées, lorsque l'angle de pivotement effectif du fémur par rapport à sa position initiale, dépasse l'angle de pivotement maximal.

8. Procédé selon l'une des revendications précédentes, **caractérisé en ce que** pour le calcul de l'axe mécanique du fémur, on calcule, à partir de la surface partielle sphérique balayée par l'élément de marquage, le centre de cette surface partielle sphérique, et l'on détermine l'axe mécanique par la ligne de liaison de ce centre avec l'articulation du genou.

9. Procédé selon la revendication 8, **caractérisé en ce que** l'on détermine tout d'abord un centre virtuel de la surface partielle sphérique par le fait que l'on calcule à partir des données de position de la surface partielle sphérique, mémorisées, approximativement un plan et ensuite une perpendiculaire à celui-ci passant par l'articulation du genou, et on suppose le centre virtuel à une distance prédéterminée de ce plan, sur la perpendiculaire, et **en ce que** l'on calcule l'axe mécanique du fémur en utilisant les données de position du centre virtuel et les données de position de la surface partielle sphérique.

10. Procédé selon la revendication 9, **caractérisé en ce que** l'on convertit par calcul les données de position de la surface partielle sphérique en utilisant les données de position du centre virtuel, sur un angle de pivotement unitaire par rapport à la position initiale, de sorte que l'on obtient en conséquence des données de position corrigées définissant un cercle commun, et **en ce que** l'on calcule comme axe mécanique du fémur, la perpendiculaire centrale de ce cercle.

11. Procédé selon l'une des revendications précédentes, **caractérisé en ce que** l'on détermine le nombre et la répartition des données de position mesurées, dans la totalité de la plage de pivotement, et, après avoir atteint un nombre prescrit et une répartition prescrite, on interrompt le relevé d'autres données de position supplémentaires.

12. Procédé selon l'une des revendications précédentes, **caractérisé en ce que** l'on représente sous forme d'image les données de position mémorisées conformément à leur répartition spatiale dans la zone de pivotement, conformément à cette répartition, de sorte que l'on peut voir, pour quelle partie de la zone de pivotement possible, quel est le nombre de données de position ayant été mémorisé.

13. Procédé selon la revendication 12, **caractérisé en ce que** sur une surface représentée sous forme d'image, on marque des zones partielles de cette surface lorsque dans une partie de la zone de pivotement du fémur, correspondant à cette zone partielle, on a relevé un nombre déterminé de données de position.

14. Procédé selon la revendication 13, **caractérisé en ce que** la surface est une surface annulaire subdivisée en segments ou secteurs.

15. Dispositif pour la mise en oeuvre du procédé des revendications 1 à 14, comprenant un système de navigation pour déterminer des données de position d'un élément de marquage fixé au fémur, et un système informatique ou de traitement de données pour calculer la position de l'axe mécanique du fémur à partir desdites données de position, **caractérisé en ce que** le système de traitement de données (22), pour calculer la position de l'axe mécanique du fémur (5), sélectionne à partir desdites données de position, celles qui se situent en-dessous un angle limite déterminé, qui est inférieur à l'angle de pivotement maximal balayé par le fémur (5).

16. Dispositif selon la revendication 15, **caractérisé en ce que** l'angle limite se situe entre 4° et 6°.

17. Dispositif selon la revendication 15 ou 16, **caractérisé en ce que** le système de traitement de données (22), pour calculer la position de l'axe mécanique du fémur (5), sélectionne à partir desdites données de position, celles qui se situent au-dessus d'un angle minimal déterminé, qui est inférieur à l'angle limite.

18. Dispositif selon la revendication 17, **caractérisé en ce que** l'angle minimal se situe au-dessus de 3°.

19. Dispositif selon l'une des revendications 15 à 18, **caractérisé en ce que** le système de traitement de données (22) ne prend en considération aucune de toutes les données de position mémorisées, lorsque l'angle de pivotement effectif du fémur (5) par rapport à sa position initiale, dépasse l'angle de pivotement maximal.

20. Dispositif selon l'une des revendications 15 à 19, **caractérisé en ce que** le système de traitement de données (22), pour le calcul de l'axe mécanique du fémur (5), calcule, à partir du centre de la surface partielle sphérique balayée par l'élément de marquage (7), le centre de cette surface partielle sphérique, et détermine l'axe mécanique par la ligne de liaison de ce centre avec l'articulation du genou (4).

21. Dispositif selon la revendication 20, **caractérisé en ce que** le système de traitement de données (22) détermine tout d'abord un centre virtuel (30) de la surface partielle sphérique par le fait qu'il calcule à partir des données de position de la surface partielle sphérique, mémorisées, approximativement un plan et ensuite une perpendiculaire à celui-ci passant par l'articulation du genou (4), et suppose le centre virtuel (30) à une distance prédéterminée de ce plan, sur la perpendiculaire, et **en ce que** le système de traitement de données (22) calcule l'axe mécanique du fémur (5) en utilisant les données de position du centre virtuel (30) et les données de position de la surface sphérique partielle.

22. Dispositif selon la revendication 21, **caractérisé en ce que** le système de traitement de données (22) convertit par calcul les données de position de la surface partielle sphérique en utilisant les données de position du centre virtuel (30), sur un angle de pivotement unitaire par rapport à la position initiale, de sorte que l'on obtient en conséquence des données de position corrigées définissant un cercle commun, et **en ce qu'**il calcule comme axe mécanique du fémur (5), la perpendiculaire centrale de ce cercle.

23. Dispositif selon l'une des revendications 15 à 22, **caractérisé en ce que** le système de traitement de données (22) détermine le nombre et la répartition des données de position mesurées, dans la totalité de la plage de pivotement, et, après avoir atteint un nombre prescrit et une répartition prescrite, interrompt le relevé d'autres données de position supplémentaires.

24. Dispositif selon l'une des revendications 21 à 23, **caractérisé en ce que** le système de traitement de données (22) représente sous forme d'image les données de position mémorisées conformément à leur répartition spatiale dans la zone de pivotement, conformément à cette répartition, de sorte que l'on peut voir, pour quelle partie de la zone de pivotement possible, quel est le nombre de données de position ayant été mémorisé.

25. Dispositif selon la revendication 24, **caractérisé en ce que** le système de traitement de données (22), sur une surface (27) représentée sous forme d'image, marque des zones partielles (28) de cette surface (27) lorsque dans une partie de la zone de pivotement du fémur (5), correspondant à cette zone partielle (28), a été relevé un nombre déterminé de données de position.

26. Dispositif selon la revendication 25, **caractérisé en ce que** la surface (27) est une surface annulaire subdivisée en segments ou secteurs (28).
